# EUROPÄISCHE PATENTANMELDUNG

(11) **EP 1 498 069 A1**
(43) Veröffentlichungstag der Anmeldung: **19.01.2005**
(21) Anmeldenummer: 04015876.8
(22) Anmeldetag: 06.07.2004
(51) Int. Cl.: A61B 5/0476, A61B 5/05

(54) **Evaluierung der Leistungsfähigkeit und Belastbarkeit für Fahrzeugführer**

(30) Priorität: 14.07.2003 DE 10331800
(71) Anmelder: DaimlerChrysler AG, 70567 Stuttgart (DE)
(72) Erfinder: Elitok, Ercan, Dr.med., 89073 Ulm (DE); Hahn, Stefan, Dipl.-Inf., 89075 Ulm (DE); Kincses, Wilhelm, Dr.rer.nat., 73732 Esslingen (DE); Schrauf, Michael, Dr., 40764 Langenfeld (DE)

(57) **Zusammenfassung**

Beschrieben wird ein Verfahren und System zur Sondierung der aktuellen Belastung bzw. Leistungs- und Aufnahmefähigkeit von Fahrzeugführern. Dabei werden kontinuierlich EEG-Muster der mentalen Aktivität in verschiedenen Frequenzbereichen aufgenommen und nach verschiedenen Kriterien ausgewertet. Auftretende Signalformen oder -veränderungen, die auf eine Beeinträchtigung der Leistungsfähigkeit hinweisen, werden so erfasst und führen je nach detektiertem Status graduell abgestuft zur Auslösung von Warnsignalen bis hin zum direkten Eingriff in die Fahrzeugsteuerung.

## Beschreibung

Die Erfindung betrifft Verfahren und Einrichtungen zur Erfassung situationsbedingter physiologischer und mentaler Konditionen eines Menschen. Insbesondere betrifft die Erfindung Systeme, bei denen in Fahrzeugen während der Fahrt eine Sondierung der aktuellen Belastung bzw. Aufnahme- oder Leistungsfähigkeit des Fahrers erfolgen kann.

Während der Fahrt ist der Fahrer unterschiedlichen Anforderungen ausgesetzt. Je nach Fahrsituation (z.B. Verkehrsdichte, Witterungseinflüsse), Zweitaufgaben (z.B. Navigieren, Aufnahme von Verkehrsdurchsagen, Ablenkung durch Mitfahrer) und Fahrereigenschaften (Alter, Erfahrung) führen diese Anforderungen in Verbindung mit den aktuellen Fahrerkapazitäten (z.B. Müdigkeit) zu verschiedenen Stufen der Arbeitsbelastung. Die objektive Kenntnis der aktuellen Belastung und Kondition des Fahrers ist zur Verbesserung der Mensch-Maschine-Interaktion von erheblicher Bedeutung und dient damit der allgemeinen Fahrsicherheit.

Üblicherweise wird die Leistungsfähigkeit einer Person bei verschiedenen Belastungsstufen über die Bewältigung von Zweitaufgaben anhand von Fragebögen ermittelt. Dieses Verfahren ist prinzipiell stark fehlerbehaftet, da die Wichtung der Aufgaben beim Ausfüllen des Fragebogens individuell stark differieren kann. Zudem beinhaltet die Methode der Zweitaufgabe stets eine Interferenz mit der Hauptaufgabe (Problem der Zuordnung der Ergebnisse). Generell ist das Verfahren nicht auf oben dargestellte "ad hoc" Belastungssituation beim Führen eines Fahrzeugs übertragbar.

Ein alternatives Verfahren nutzt zur Erfassung der aktuellen mentalen Kondition einer Person die bei unterschiedlicher Aktivität auftretende Variabilität von Hirnströmen. Ein auf diesem Prinzip beruhendes System zur Analyse von Informationen über das Wohlbefinden und den Wachheitszustand eines Fahrzeugführers ist beschrieben in der Patentschrift US5884626 (Kuroda et al.). Bei dieser Anordnung werden Fluktuationen spezieller Hirnströme in einem bestimmten Frequenzband mittels Elektroenzephalogramm (EEG) aufgezeichnet und auf signifikante Veränderungen analysiert. Entsprechend der Zielsetzung (Wachheits-/Müdigkeits-Sondierung) werden hier nur Frequenzanteile.im Bereich 8 bis 13 Hertz (entspricht dem sog. α-Band) erfasst, da in diesem Frequenzintervall Ermüdungserscheinungen besonders deutliche Signalveränderungen auslösen.

Diese Technik weist verschiedene Nachteile und Einschränkungen auf. Zum einen ist es durch das vorgegebene Frequenzband prinzipiell nicht möglich, eine zeitliche Auflösung von weniger als 100 ms zu erhalten (= Dauer einer 10Hz Sinusschwingung). Damit können verschiedene andere mögliche Einflüsse auf den aktuellen Belastungsgrad nicht detektiert werden. Die Signal-Aktivität im α-Band liefert zudem nur ein grobes Maß für den allgemeinen Wachheitszustand (= geringe α-Aktivität) bzw. Müdigkeit (= hohe α-Aktivität) einer Person. Mit diesem Verfahren ist es nicht möglich, zwischen den verschiedenen Komponenten eines Belastungszustands zu unterscheiden (frühe perzeptuelle, mittlere handlungsrelevante und späte emotionale Komponenten).

Die Erfindung geht aus von der o.g. US 5884626 als nächstliegendem Stand der Technik. Ihr liegt die Aufgabe zugrunde, ein verbessertes Verfahren und System zur Erfassung mentaler Konditionen und Belastungswerte eines Fahrzeugführers zu entwickeln, das die genannten Nachteile weitgehend überwindet und darüber hinaus weitere Vorzüge aufweist.

Diese Aufgabe wird bei einem Verfahren mit den Merkmalen des Oberbegriffs des Anspruchs 1 gelöst durch die kennzeichnenden Merkmale des Anspruchs 1. Die Merkmale einer entsprechenden Vorrichtung nach dem erfindungsgemäßen Verfahren sind in Anspruch 8 aufgeführt. Weitere Einzelheiten der Erfindung und Vorzüge verschiedener Ausführungsformen ergeben sich aus den Merkmalen der Unteransprüche.

Das erfindungsgemäße Verfahren und die entsprechende Vorrichtung wird im folgenden anhand einer bevorzugten Ausführungsform beschrieben, wobei Bezug genommen wird auf die Abbildungen und den darin aufgeführten Bezugsziffern.
Es zeigt:
- Figur 1:: charakteristische Hirnstromkurven bei epileptischen Anfällen
- Figur 2:: Messbare Veränderungen von Signalen bei stimulierten Reizen

Die Erfindung berücksichtigt die Tatsache, dass neben Müdigkeit und Wohlbefinden auch andere Einflüsse die Leistungsfähigkeit einer Person beinträchtigen können. Verschiedene Stufen der aktuellen Belastung und Aufnahmefähigkeit korrelieren mit bestimmten Phasen der Hirnaktivität, diese gehen mit Veränderungen von Hirnstromanteilen einher, die nicht auf das α-Band allein beschränkt sind. Signale in tieferen und höheren Frequenzbändern können daher wertvolle zusätzliche Informationen über die mentale Kondition und Aufnahmefähigkeit einer Person liefern. Dazu gehören auch Beeinträchtigung der Leistungsfähigkeit durch Krankheit oder auch durch Medikamente oder Drogen.

Generell unterscheidet man in der Neurologie bei Hirnstrommessungen unterschiedliche Frequenzbänder:
Das Sub-δ-Band mit einem Frequenzbereich von 0.15 bis 0.5 Hz,
das δ-Band mit einem Frequenzbereich von 0.5 bis 3.5 Hz,
das θ-Band mit einem Frequenzbereich von 3.5 bis 8 Hz,
das α-Band mit einem Frequenzbereich von 8 bis 13 Hz,
das β-Band mit einem Frequenzbereich von 13 bis 30 Hz, sowie Frequenzanteile oberhalb des β-Bandes (also über 30 Hz).

Signale und deren Veränderungen in den verschiedenen Bändern können nach heutigem medizinischem Wissensstand mit hoher Genauigkeit unterschiedlichen Einflüssen, mentalen Reaktionen und Beeinträchtigungen zugeordnet werden.

Neben dem bereits im Stand der Technik erwähntem α-Band als Informationsträger über den Wachheitszustand einer Person, liefern Frequenzanteile oberhalb dieses Bandes (β-Band und darüber) Informationen über Beeinträchtigung der mentalen Funktionen durch Drogen (z.B. Alkohol) oder zentral wirksame Medikamente (z.B. Hustenblocker mit hoher Codein-Dosierung). Eine solche Beeinträchtigung zu sondieren, ist für die Fahrsicherheit von hoher Bedeutung.

Das direkt unterhalb des α-Bands angrenzende θ-Band liefert Informationen anderer Art, die jedoch ebenfalls große Bedeutung für die Fahrsicherheit haben: Einschlafphasen korrelieren nämlich mit signifikanten Veränderungen der Signale im θ-Band. Spontan auftretende Einschlafvorgänge können bei hoher Ermüdung, aber auch krankheitsbedingt (sog. "Narkolepsie") auftreten. Durch kontinuierliche Sondierung dieses Frequenzbereichs könnte das Eintreten eines (häufig für Unfälle verantwortlichen) sog. "Sekundenschlafs" relativ frühzeitig erkannt werden und durch Auslösen von Wecksignalen (akustisch, optisch, taktil) oder bei kritischen Situation u.U. durch direkten Eingriff in die Fahrzeugsteuerung (Einleiten eines Bremsvorgangs) die Unfallgefahr reduziert werden.

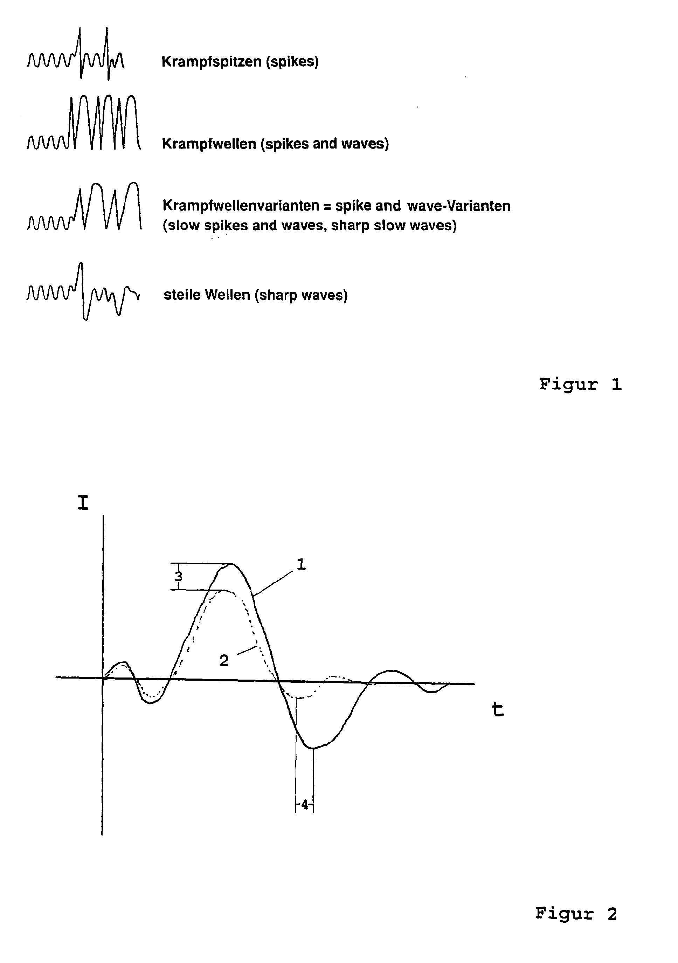

Eine andere Art krankheitsbedingter Beeinträchtigung mentaler Belastungs- bzw. Aufnahmefähigkeit sind z.B. epileptische Anfälle, die in verschiedener Ausprägung spontan auftreten können. Damit einhergehen stets charakteristische Veränderungen der Hirnstromaktivitäten, insbesondere Veränderungen im δ-und Sub-δ-Band. Typische Signalformen sind in der Figur 1 dargestellt. Man unterscheidet verschiedene Anfallstärken, wobei jedoch selbst ein "leichter" Anfall im Straßenverkehr ein hohes Gefahrenpotenzial bedeutet, da hier bereits typischerweise Wahrnehmungsstörungen auftreten. Eine sogenannte "Aura" ist der medizinische Begriff für eine kurzzeitige und in der Regel nur auf eine lokale Hirnregion begrenzte Funktionsstörung "(Fokaler" Anfall), die mit deutlich veränderter Wahrnehmung der Umwelt einhergeht und damit die Fahrtüchtigkeit stark einschränkt. Auch die sog. "Absencen" treten ohne subjektiv spürbare Vorwarnung spontan auf. Bei dieser Anfallart ist für eine Zeit von typischerweise unter 20 Sekunden jegliche Wahrnehmung vollständig unterbrochen, wobei diese Bewusstseinsstörung abrupt einsetzt und ebenso plötzlich endet. Ein starker Anfall (*grand mal*) schließlich erfasst mehrere Hirnregionen simultan ("Generalisierter" Anfall). Ein bekannter Auslöser für diese Anfallart sind periodische Lichtänderungen (Flackerlicht). Eine solche Situation könnte beispielsweise auftreten beim Fahren auf einer Allee, wo die Baumreihen am Straßenrand bei seitlicher Sonneneinstrahlung zu eben diesem flackernden Lichtreiz führen können. Im EEG messbare "Vorboten" eines generalisierten Anfalls sind synchronisierte Signale.

In einer erweiterten Ausführung der Erfindung werden Stimuli verwendet, die dann spezielle mentale Aktivität auslösen. Geeignet sind beispielsweise optische Muster (z.B. Piktogramme, Bilder, Videosequenz) und/oder akustische Signale (z.B. Geräusche, Sprache, Tonfolgen, Musiksequenz) aber auch taktile Reize (z.B. leichte Vibrationen). Stimuli dieser Art evozieren Hirnstrompotenziale mit charakteristischem zeitlichem Verlauf. In Figur 2 ist ein Beispiel für ein evoziertes Potenzial schematisch dargestellt. Das bei voller Leistungsfähigkeit generierte Signal (1) verändert sich, wenn Beeinträchtigungen durch Ermüdung, Überbelastung, Medikamente etc. auftreten. Durch Vergleich von Unterschieden zwischen normalem evozierten Potenzial (= Referenzmuster) mit einem aktuell aufgenommenen Signal (2), z.B. bezüglich Amplitudenhöhe (3) und/oder zeitlicher Verschiebungen (4) der Kurvenmaxima, lassen sich Aussagen über die momentane mentale Kondition des Fahrzeugführers ableiten. So ist bekannt, dass eine Abnahme der Fähigkeit, Informationen zu verarbeiten, mit Veränderungen evozierter Potenziale im Zeitbereich von 300 Millisekunden (sog. "P300") einhergehen.

Einflüsse oder Störungen, die die Leistungs- bzw. Aufnahmefähigkeit des Fahrzeugführers beeinträchtigen können, lassen sich erfindungsgemäß durch entsprechend differenzierte "Multi-Band"-Signalanalyse, d.h. über einen sehr breiten Frequenzbereich, identifizieren. Die kontinuierliche Ableitung der Hirnstromaktivität an mehreren Punkten der Kopfoberfläche (beispielsweise mittels 32 Elektroden) ermöglicht dabei eine Kartierung bzw. Verteilung der aktuellen Aktivitäten und Zuordnung einzelner Anteile zu verschiedenen Hirnregionen. Erfindungsgemäß umfasst dabei die Signalauswertung insbesondere:
- kontinuierliche Überwachung der Intensitätsverläufe in den verschiedenen Bändern,
- Identifizierung charakteristischer Signalformen und korrelierter Signalveränderungen (Auftreten synchroner Signale bzw. Signalveränderungen an verschiedenen Abtastelektroden bzw. in verschiedenen Bändern),
- Vergleich der detektierten Signalformen und -veränderungen mit entsprechenden gespeicherten Daten (z.B. Referenzmustern, -fluktuationen).

Diese Auswertung kann mittels üblicher digital-elektronischer Komponenten erfolgen, die beispielsweise Teil eines Mikroprozessorsystems sind. Ein computergestütztes System zur Analyse von EEG-Mustern ist z.B. beschrieben in der US 5447166.

Um während der Fahrt kontinuierlich die aktuelle mentale Kondition, Belastung bzw. Aufnahmefähigkeit des Fahrers zu sondieren, müssen Hirnstromsignale permanent aufgenommen und auf neurologische Kennwerte analysiert werden. Übliche EEG-Geräte verwenden zur Ableitung der auszuwertenden Gehirnströme Elektroden, die - z.B. mittels elektrolythaltigen Gels - in direktem elektrischen Kontakt mit der Kopfhaut stehen. Dabei sind hier entsprechende Anforderungen an den Tragekomfort zu stellen, um Beeinträchtigungen der Fahrzeugführung auszuschließen. Ein leichtes, tragbares EEG-Gerät ist beispielsweise beschrieben in der Zeitschrift *"Medical & Biological Engineering & Computing"* 1994, p 459-461, H. Iguchi et al.: *"Wearable Electroencephalograph System With Preamplified E-lectrodes".* Weiterentwickelte Systeme mit hochsensitiven Felddetektoren können ebenfalls eingesetzt werden. Dabei werden kontaktlos die von den Gehirnströmen erzeugten elektrischen und/oder magnetischen Felder detektiert und ausgewertet. Kontaktlose EEG-Messungen dieser Art sind beispielsweise mit SQUID-Detektoren durchgeführt worden. Eine solche Vorrichtung bietet je nach Positionierung der feldaufnehmenden Detektoren (z.B. im Bereich der Kopfstütze) den Vorteil eines hohen Komforts und entsprechend hoher Akzeptanz.

Das erfindungsgemäße Verfahren und die entsprechenden Vorrichtungen sind speziell für den Einsatz in Kraftfahrzeugen sehr gut geeignet, da die erforderlichen Komponenten (Detektoren, Verstärker, Mikroprozessorsystem usw.) in heutiger Integrationstechnik sehr klein und leicht ausgeführt werden können und auch nur einen geringen Energieverbrauch aufweisen. Das System ist dabei sehr flexibel und kann sehr leicht auf unterschiedliche Personen eingestellt werden. Individuelle Daten verschiedener Fahrzeugführer können gespeichert, neu aufgenommen, modifiziert oder gelöscht werden. Ebenso können Einstellungen verschiedenster Art jederzeit modifiziert werden.

Grundsätzlich leistet das erfindungsgemäße Verfahren und System einen Beitrag zur Sicherheit im Straßenverkehr. Ist die Leistungsfähigkeit des Fahrzeugführers beeinträchtigt, so können stufenweise entsprechende Reaktionen zur Unfallvermeidung einleitet werden: von Pausenempfehlung bei einsetzender Ermüdung, über Warnsignale bei deutlicher Leistungsverringerung bis hin zu direktem Eingriff in die Fahrzeugführung bei kritischer Wahrnehmungsstörung.

## Patentansprüche

1. Verfahren zur Erfassung mentaler Konditionen (Belastbarkeit, Aufnahme- und Leistungsfähigkeit) einer Person während des Führens eines Fahrzeugs durch Analyse von Hirnstromsignalen,
**dadurch gekennzeichnet,**
**dass** Hirnstromsignale des Fahrzeugführers simultan in mehreren Frequenzbändern (z.B. Sub-θ-Band, θ-Band, δ-Band, α-Band, β-Band) abgeleitet und analysiert werden.

2. Verfahren nach Anspruch 1,
**dadurch gekennzeichnet,**
**dass** Hirnstromsignale in verschiedenen Frequenzbändern selektiv auf Intensitätsveränderungen analysiert werden.

3. Verfahren nach Anspruch 1 oder 2,
**dadurch gekennzeichnet,**
**dass** Hirnstromsignale auf charakteristische Signalformen analysiert werden.

4. Verfahren nach Anspruch 1, 2 oder 3,
**dadurch gekennzeichnet,**
**dass** Hirnstromsignale in verschiedenen Frequenzbändern auf Korrelationen (z.B. Auftreten synchroner Signale bzw. Signalveränderungen) analysiert werden.

5. Verfahren nach einem der Ansprüche 1 bis 4,
**dadurch gekennzeichnet,**
**dass** die Analyse der Hirnstromsignale einen Vergleich mit gespeicherten Daten (z.B. Referenzmustern, Signifikanzwerte für Korrelationen, charakteristische Fluktuationsparameter etc.) beinhaltet.

6. Verfahren nach einem der Ansprüche 1 bis 5,
**dadurch gekennzeichnet,**
**dass** durch vorgegebene Reize (visuell, akustisch, taktil) Hirnstromsignale stimuliert werden.

7. Verfahren nach einem der Ansprüche 1 bis 6,
**dadurch gekennzeichnet,**
**dass** bei Erfassung einer Beeinträchtigung der Belastbarkeit bzw. Aufnahme- und Leistungsfähigkeit graduell abgestuft Warn- oder Steuerungsprozesse ausgelöst werden.

8. Vorrichtung zur Durchführung des Verfahrens nach einem der Ansprüche 1 bis 7,
**dadurch gekennzeichnet,**
**dass** die Mittel zur Speicherung und Analyse der EEG-Signale als Mikroprozessorsystem ausgeführt sind.

9. Vorrichtung nach Anspruch 8,
**dadurch gekennzeichnet,**
**dass** Mittel zur optischen, akustischen und/oder taktilen Stimulierung der Person vorhanden sind.

10. Vorrichtung nach Anspruch 8 oder 9,
**dadurch gekennzeichnet,**
**dass** die Mittel zur Aufnahme der EEG-Signale als kontaktlose Felddetektoren ausgebildet sind.
